# EUROPÄISCHE PATENTSCHRIFT

(11) **EP 2 336 212 B1**
(45) Veröffentlichungstag und Bekanntmachung des Hinweises auf die Patenterteilung: **07.03.2012**
(21) Anmeldenummer: 09015401.4
(22) Anmeldetag: 12.12.2009
(51) Int. Cl.: C08G 18/10, C09D 175/04, C09D 175/12, C09J 175/04, C09J 175/12, A61L 24/04, C09J 7/04

(54) **Klebstoffverbundsystem zum Abdecken, Verschliessen oder Verkleben von Zellgewebe**
Compound adhesive system for covering, sealing or glueing cell tissue
Système composite de colle destiné à recouvrir, fermer ou coller des tissus cellulaires

(43) Veröffentlichungstag der Anmeldung: 22.06.2011
(73) Patentinhaber: Bayer MaterialScience AG, 51368 Leverkusen (DE)
(72) Erfinder: Dörr, Sebastian, Dr., 40597 Düsseldorf (DE); Heckroth, Heike, Dr., 51519 Odenthal (DE); Eggert, Christoph, Dr., 50733 Köln (DE)

(56) Entgegenhaltungen:
- EP-A1- 2 011 808
- WO-A1-90/06330
- DE-A1- 3 903 796

## Beschreibung

Die vorliegende Erfindung betrifft ein Klebstoffverbundsystem. Weitere Gegenstände der Erfindung sind ein Verfahren zur Herstellung des Klebstoffverbundsystems, ein nach dem Verfahren erhältliches Klebstoffverbundsystem, ein Klebstoffverbundsystem zur Verwendung als Mittel zum Abdecken, Verschließen oder Verkleben von Zellgewebe und die Verwendung des Klebstoffverbundsystems zur Herstellung eines Mittels zum Abdecken, Verschließen oder Verkleben von Zellgewebe.

Aus der EP 2 011 808 A1 sind Gewebekleber bekannt, die auf einem hydrophilen 2-Komponenten-Polyurethan-System beruhen. Diese Gewebekleber können zum Abdecken, Verschließen oder Verkleben von Zellgewebe und insbesondere zum Verkleben von Wunden eingesetzt werden. Die beschriebenen Gewebekleber zeichnen sich dabei durch eine starke Bindung zum Gewebe, eine hohe Flexibilität der erhaltenen Fügenaht, eine leichte Applizierbarkeit, eine in einem weiten Bereich einstellbare Aushärtungszeit und eine hohe Biokompatibilität aus.

Allerdings treten bei der Verwendung der bekannten Gewebekleber auch bestimmte Probleme auf. So kann es aufgrund der Hydrophilie der Polyurethan-Systeme bei längerer Exposition mit Wasser zu einer Quellung des Gewebeklebers kommen. Hierdurch wird die Haftung des Gewebeklebers zum Gewebe verringert, was sich insgesamt negativ auf die Haltbarkeit der Verklebung auswirken kann.

Aufgabe der vorliegenden Erfindung war es daher ein Klebstoffverbundsystem bereit zu stellen, das zur Herstellung einer leicht applizierbaren, biokompatiblen, stark an Gewebe anhaftenden elastischen Verklebung verwendet werden kann, die auch bei einer längeren Exposition mit Wasser nicht quillt und somit auch unter diesen Bedingungen dauerhaft haltbar ist.

Diese Aufgabe ist durch ein Klebstoffverbundsystem umfassend eine Kleberschicht aus einem Gewebekleber und eine flächig auf der Kleberschicht aufgebrachte Schutzschicht, bei dem der Gewebekleber auf hydrophilen Polyurethanpolymeren basiert und die Schutzschicht wasserundurchlässig ist, gelöst.

Als "wasserundurchlässig" im Sinne der vorliegenden Erfindung wird eine Schutzschicht bezeichnet, die eine darunterliegende Kleberschicht für eine Zeit von mindestens 30 Minuten vor Quellung schützt, wenn das Klebstoffverbundsystem aus Kleberschicht und Schutzschicht in ein Wasserbad mit einer Temperatur von zu bis 40°C eingetaucht wird.

Die wasserundurchlässige Schicht zeichnet sich bevorzugt dadurch aus, dass bei Lagerung einer derartigen Schicht als freier Film mit einer Schichtstärke von 100 Micrometem in einem Überschuss entmineralisierten Wassers bei 23°C für einen Zeitraum von 2 Stunden die Massen an aufgenommenem Wasser, bezogen auf die Ausgangsmasse des Films, unter 100%, bevorzugt unter 50%, besonders bevorzugt unter 20% und ganz besonders bevorzugt unter 10% beträgt.

Der Gewebekleber umfasst
A) isocyanatfunktionelle Prepolymere erhältlich aus
   A1) aliphatischen Isocyanaten und
   A2) Polyolen mit zahlenmittleren Molekulargewichten von ≥ 400 g/mol und mittleren OH-Funktionalitäten von 2 bis 6,
B) aminofunktionelle Asparaginsäureester der allgemeinen Formel (I) 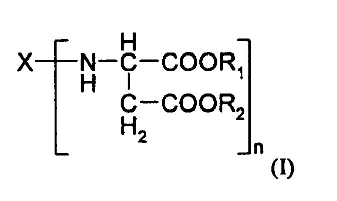 bei der
   - X: ein n-wertiger organischer Rest ist, der durch Entfernung einer primären Aminogruppen eines n-wertigen Amins erhalten wird,
   - R₁, R₂: gleiche oder verschiedene organische Reste sind, die keinen Zerewitinoff- aktiven Wasserstoff aufweisen und
   - n: eine ganze Zahl von mindestens 2 ist.
   und / oder
C) Umsetzungsprodukte isocyanatfunktioneller Prepolymere A) mit Asparaginsäureestem B).

Der vorstehend genannte Gewebekleber zeichnet sich durch eine starke Bindung zum Gewebe, eine hohe Flexibilität der erhaltenen Fügenaht, eine leichte Applizierbarkeit, eine in einem weiten Bereich einstellbare Aushärtungszeit und eine hohe Biokompatibilität aus.

Zur Definition von Zerewitinoff-aktivem Wasserstoff wird auf den entsprechenden Eintrag "aktiver Wasserstoff" in Römpp Chemie Lexikon, Georg Thieme Verlag Stuttgart verwiesen. Bevorzugt werden unter Gruppen mit Zerewitinoff-aktivem Wasserstoff OH, NH oder SH verstanden.

Als Isocyanate A1) können beispielsweise monomere aliphatische oder cycloaliphatische Di- oder Triisocyanate wie 1,4-Butylendiisocyanat (BDI), 1,6-Hexamethylendiisocyanat (HDI), Isophorondiisocyanat (IPDI), 2,2,4- und/oder 2,4,4-Trimethylhexamethylendüsocyanat, die isomeren Bis-(4,4'-isocyanatocyclohexyl)methane oder deren Mischungen beliebigen Isomerengehalts, 1,4-Cyclohexylendiisocyanat, 4-Isocyanatomethyl-1,8-octandiisocyanat (Nonan-triisocyanat), sowie Alkyl-2,6-diisocyanatohexanoate (Lysindiiocyanat) mit C1-C8-Alkylgruppen eingesetzt werden.

In einer besonders bevorzugten Ausführungsform wird ausschließlich Hexamethylendiisocyanat eingesetzt.

Neben den vorstehend genannten monomeren Isocyanaten können auch deren höhermolekulare Folgeprodukte mit Uretdion-, Isocyanurat-, Urethan-, Allophanat-, Biuret-, Iminooxadiazindion- oder Oxadiazintrionstruktur sowie deren Mischungen eingesetzt werden.

Vorzugsweise können die Isocyanate A1) ausschließlich aliphatisch oder cycloaliphatisch gebundene Isocyanatgruppen aufweisen.

Die Isocyanate oder Isocyanatmischungen A1) haben bevorzugt eine mittlere NCO-Funktionalität von 2 bis 4, besonders bevorzugt 2 bis 2,6 und ganz besonders bevorzugt 2 bis 2,4.

Als Polyole A2) können grundsätzliche alle dem Fachmann an sich bekannten Polyhydroxyverbindungen mit 2 oder mehr OH-Funktionen pro Molekül eingesetzt werden. Dies können beispielsweise Polyesterpolyole, Polyacrylatpolyole, Polyurethanpolyole, Polycarbonatpolyole, Polyetherpolyole, Polyesterpolacrylatpolyole, Polyurethanpolyacryl-atpolyole, Polyurethanpolyesterpolyole, Polyurethanpolyetherpolyole, Polyurethanpolycarbonat-polyole, Polyesterpolycarbonatpolyole oder deren beliebige Mischungen untereinander sein.

Die Polyole A2) haben bevorzugt eine mittlere OH-Funktionalität von 3 bis 4

Die Polyole A2) haben ferner bevorzugt ein zahlenmittleres Molekulargewicht von 400 bis 20000 g/mol, besonders bevorzugt von 2000 bis 10000 g/mol und ganz besonders bevorzugt von 4000 bis 8500.

Besonders bevorzugte Polyetherpolyole sind Polyalkylenoxid-Polyether auf Basis von Ethylenoxid und gegebenenfalls Propylenoxid.

Diese Polyetherpolyole basieren bevorzugt auf di- oder höherfunktionellen Startermolekülen wie zwei- oder höherfunktionellen Alkoholen oder Aminen.

Beispiele solcher Starter sind Wasser (als Diol aufgefasst), Ethylenglykol, Propylenglykol, Butylenglykol, Glycerin, TMP, Sorbit, Pentaerythrit, Triethanolamin, Ammoniak oder Ethylendiamin.

Bevorzugt ist auch, wenn die Polyole A2) Polyalkylenoxid-Polyether sind, die insbesondere einen Gehalt an Ethylenoxid-basierten Einheiten von 60 bis 90 Gew.-%, bezogen auf die insgesamt enthaltene Mengen an Alkylenoxideinheiten, aufweisen.

Bevorzugte Polyesterpolyol sind Polykondensate aus Di- sowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Butandiol(1,4), Neopentylglykol und Hydroxypivalinsäureneopentylglykolester bevorzugt sind. Daneben können auch Polyole wie Trimethylolpropan, Glycerin, Erythrit, Pentaerythrit, Trimetylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbemsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und Phthalsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können hydroxylgruppenaufweisende Polycarbonate, bevorzugt Polycarbonatdiole, mit zahlenmittleren Molekulargewichten Mn von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art in Frage.

Zur Herstellung des Prepolymers A) können Isocyanate A1) mit Polyolen A2) bei einem NCO/OH-Verhältnis von bevorzugt 4:1 bis 12:1, besonders bevorzugt von 8:1 umgesetzt werden. Anschließend kann der Anteil an nicht umgesetzten Isocyanaten A1) mittels geeigneter Methoden abgetrennt. Üblicherweise wird hierfür die Dünnschichtdestillation eingesetzt, wobei restmonomerenarme Produkte mit Restmonomergehalten von weniger als 1 Gew.-%, bevorzugt weniger als 0,5 Gew.-%, ganz besonders bevorzugt weniger als 0,1 Gew.-% erhalten werden.

Gegebenenfalls können während der Herstellung Stabilisatoren wie Benzoylchlorid, I-sophthaloylchlorid, Dibutylphosphat, 3-Chlorpropionsäure oder Methyltosylat zugesetzt werden.

Die Reaktionstemperatur beträgt dabei insbesondere 20 bis 120°C, bevorzugt 60 bis 100°C.

Bevorzugt aminofunktionelle Asparaginsäureester bei denen in der Formel (I):
R1, R2 gleiche oder verschiedene gegebenenfalls verzweigte oder cyclische organische Reste, die keinen Zerewitinoff-aktiven Wasserstoff aufweisen, mit 1 bis 20, bevorzugt 1 bis 10 Kohlenstoffatomen, besonders bevorzugt Methyl- oder Ethylgruppen, ,
   - n: eine ganze Zahl von 2 bis 4 und
   - X: ein n-wertiger organischer gegebenenfalls verzweigter oder cyclischer organischer Rest mit 2 bis 20, bevorzugt 5 bis 10 Kohlenstoffatomen, der durch Entfernung einer primären Aminogruppen eines n-wertigen primären Amins erhalten wird
sind.

Selbstverständlich können auch Gemische von mehreren Asparaginsäureestern verwendet werden, so dass n in der Formel (I) auch einen nicht ganzzahligen Mittelwert darstellen kann.

Die Herstellung der aminofunktionellen Polyasparaginsäureester B1) kann in bekannter Weise durch Umsetzung der entsprechenden primären mindestens difunktionellen Amine X(NH₂)ₙ mit Malein- oder Fumarsäureestern der allgemeinen Formel (II) 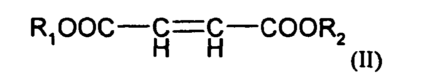 erfolgen. Bevorzugte Malein- oder Fumarsäureester sind Maleinsäuredimethylester, Maleinsäurediethylester, Maleinsäuredibutylester und die entsprechenden Fumarsäureester.

Bevorzugte primäre mindestens difunktionelle Amine X(NH₂)ₙ sind Ethylendiamin, 1,2-Diaminopropan, 1,4-Diaminobutan, 1,3-Diaminopentan, 1,5-Diaminopentan, 2-Methyl-1,5-Diaminopentan, 1,6-Diaminohexan, 2,5-Diamino-2,5-dimethylhexan, 2,2,4- und/oder 2,4,4-Trimethyl-1,6-diaminohexan, 1,11-Diaminoundecan, 1,12-Diaminododecan, 1-Amino-3,3,5-trimethyl-5-aminomethyl-cyclohexan, 2,4-und/oder 2,6-Hexahydrotoluylendiamin, 2,4'-und/oder 4,4'-Diamino-dicyclohexylmethan, 3,3'-Dimethyl-4,4'-diamino-dicyclohexylmethan, 2,4,4'-Triamino-5-methyl-dicyclohexylmethan und Polyetheramine mit aliphatisch gebundenen primären Aminogruppen mit einem zahlenmittleren Molekulargewicht Mn von 148 bis 6000 g/mol.

Besonders bevorzugte primäre mindestens difunktionelle Amine sind 1,3-Diaminopentan, 1,5-Diaminopentan, 2-Methyl-1,5-diaminopentan, 1,6-Diaminohexan, 1,13-Diamino-4,7,10-trioxatridecan. Ganz besonders bevorzugt ist 2-Methyl-1,5-diaminopentan.

In einer bevorzugten AusfUhrungsfbrm sind R1 = R2 = Ethyl, wobei X auf 2-Methyl-1,5-diaminopentan als n-wertigem Amin basiert.

Die Herstellung der aminofunktionellen Asparaginsäureester B1) aus den genannten Ausgangsmaterialien erfolgt beispielsweise nach DE-A 69 311 633, bevorzugt innerhalb des Temperaturbereichs von 0 bis 100 °C, wobei die Ausgangsmaterialien in solchen Mengenverhältnissen eingesetzt werden, dass auf jede primäre Aminogruppe mindestens eine, vorzugsweise genau eine olefinische Doppelbindung entfällt, wobei im Anschluss an die Umsetzung gegebenenfalls im Überschuss eingesetzte Ausgangsmaterialien destillativ abgetrennt werden können. Die Umsetzung kann in Substanz oder in Gegenwart geeigneter Lösungsmittel wie Methanol, Ethanol, Propanol oder Dioxan oder Gemischen derartiger Lösungsmittel erfolgen.

Um das mittlere Equivalentgewicht der insgesamt zur Prepolymervernetzung eingesetzten Verbindungen bezogen auf die NCO-reaktiven Gruppen weiter zu reduzieren, ist es möglich zusätzlich zu den in B1) eingesetzten Verbindungen auch die amino- oder hydroxyfunktionellen Umsetzungsprodukte isocyanatfunktioneller Prepolymere mit Asparaginsäureestern in einer separaten Vorreaktion herzustellen und dann als höhermolekulare Härterkomponente C) einzusetzen.

Bevorzugt werden bei der Vorverlängerung Verhältnisse von isocyanatreaktiven Gruppen zu Isocyanatgruppen von 50 zu 1 bis 1,5 zu 1, besonders bevorzugt von 15 zu 1 bis 4 zu 1 eingesetzt.

Das dafür einzusetzende isocyanatfunktionelle Prepolymer kann dabei demjenigen der Komponente A) entsprechen oder aber auch abweichend aus den Komponenten, wie sie als mögliche Bestandteile der isocyanatfunktionellen Prepolymere im Rahmen dieser Anmeldung gelistet sind, aufgebaut sein.

Die erfindungsgemäßen 2-komponentigen Klebstoffsysteme werden durch Mischung des Prepolymers mit der Härterkomponente B) bzw. C) erhalten. Das Verhältnis von NCO-reaktiven NH-Gruppen zu freien NCO-Gruppen beträgt bevorzugt 1:1,5 bis 1:1, besonders bevorzugt 1:1.

In Weiterbildung der Erfindung ist vorgesehen, dass der Gewebekleber keinen Asparaginsäureester B) sondern ausschließlich Umsetzungsprodukte C) umfasst.

Die Kleberschicht kann auch zusätzlich einen oder mehrere Wirkstoffe enthalten. Bei den Wirkstoffen kann es sich insbesondere um solche Substanzen handeln, die die Wundheilung unterstützen.

Gemäß einer weiteren bevorzugten Ausführungsform der Erfindung weist die Schutzschicht eine Bruchdehnung von 100%, bevorzugt von ≥ 200% auf. Eine solche Schutzschicht ist besonders verformbar und korrespondiert in dieser Hinsicht insbesondere gut mit den mechanischen Eigenschaften einer Polyurethan-Kleberschicht.

Die Bruchdehnung wird nach DIN EN ISO 527-1 bestimmt.

Besonders bevorzugt ist auch, wenn die Schutzschicht einen 100%-Modul von 0,5 bis 20 MPa, bevorzugt von 1 bis 15 MPa, besonders bevorzugt von 2 bis 10 MPa aufweist. Derartige Schutzschichten sind elastisch, so dass eine hohe Gesamtelastizität des Klebstoffverbundsystems resultiert, wenn auch die Kleberschicht entsprechende mechanische Eigenschaften hat. Besondere Vorteile sind also vor allem dann geben, wenn dass Klebstoffverbundsystem eine Polyurethan-basierte Kleberschicht aufweist.

Der 100%-Modul wird nach DIN EN ISO 527-1 bestimmt.

Die Schutzschicht kann insbesondere auf Polymeren basieren.

Bei den Polymeren kann es sich bevorzugt um Polyurethane, Polyester, Poly(meth)acrylate, Polyepoxide, Polyvinylacetate, Polyethylene, Polystyrole, Polybutadiene, Polyvinylchloride und / oder entsprechenden Copolymerisaten, bevorzugt Polyacrylate und / oder Polyurethane handeln.

Besonders bevorzugt sind die Polymere Polyurethane, die nach einem Prepolymerisationverfahren erhältlich sind, bei dem
a) isocyanatfunktionelle Prepolymere aus
   a1) organischen Polyisocyanaten
   a2) polymeren Polyolen mit zahlenmittleren Molekulargewichten von 400 bis 8000 g/mol, vorzugsweise 400 bis 6000 g/mol und besonders bevorzugt von 600 bis 3000 g/mol, und OH-Funktionalitäten von 1,5 bis 6, bevorzugt 1,8 bis 3, besonders bevorzugt von 1,9 bis 2,1, und
   a3) gegebenenfalls hydroxyfunktionellen Verbindungen mit Molekulargewichten von 62 bis 399 g/mol hergestellt werden, und
b) die freien NCO-Gruppen der Prepolymeren aus a) dann ganz oder teilweise mit
   b1) aminofunktionellen und/oder hydroxyfunktionellen Verbindungen mit Molekulargewichten von 32 bis 1000 g/mol, bevorzugt von 32 bis 400 g/mol
unter Kettenverlängerung umgesetzt werden.

Geeignete Polyisocyanate a1) sind aliphatische, aromatischen oder cycloaliphatische Polyisocyanate einer NCO-Funktionalität von größer oder gleich 2.

Beispiele solcher Polyisocyanate sind 1,4-Butylendiisocyanat, 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)methane oder deren Mischungen beliebigen Isomerengehalts, 1,4-Cyclohexylendiisocyanat, 4-Isocyanatomethyl-1,8-octandiisocyanat (Nonantriisocyanat), 1,4-Phenylendiisocyanat, 2,4- und/oder 2,6-Toluylendiisocyanat, 1,5-Naphthylendiisocyanat, 2,2'-und/oder 2,4'- und/oder 4,4'-Diephenylmethandüsocyanat, 1,3- und/oder 1,4-Bis-(2-isocyanato-prop-2-yl)-benzol (TMXDI), 1,3-Bis(isocyanato-methyl)benzol (XDI) sowie Alkyl-2,6-diisocyanatohexanoate (Lysindiisocyanate) mit C1-C8-Alkylgruppen.

Neben den vorstehend genannten Polyisocyanaten können auch modifizierte Diisocyanate die eine Funktionalität ≥ 2 aufweisen, mit Uretdion-, Isocyanurat-, Urethan-, Allophanat-, Biuret-, Iminooxadiazindion- oder Oxadiazintrionstruktur sowie Mischungen aus diesen anteilig eingesetzt werden.

Bevorzugt handelt es sich um Polyisocyanate oder Polyisocyanatgemische der vorstehend genannten Art mit ausschließlich aliphatisch oder cycloaliphatisch gebundenen Isocyanatgruppen oder Mischungen aus diesen und einer mittleren NCO-Funktionalität der Mischung von 2 bis 4, bevorzugt von 2 bis 2,6 und besonders bevorzugt von 2 bis 2,4. In ganz besonders bevorzugten Ausfiihrungsformen handelt es sich um difunktionelle Isocyanatbausteine, bevorzugt difunktionelle aliphatische Isocyanatbausteine.

Besonders bevorzugt werden als Polyisocyanate a1) Hexamethylendiisocyanat, Isophorondiisocyanat oder die isomeren Bis-(4,4'-isocyanatocyclohexyl)methane sowie Mischungen der vorgenannten Diisocyanate eingesetzt. In einer ganz besonders bevorzugten Ausführungsform wird eine Mischung aus Hexamethylendüsocyanat und Isophorondüsocyanat eingesetzt.

Als polymere Polyole a2) werden Verbindungen mit einem zahlenmittleren Molekulargewicht Mₙ von 400 bis 8000 g/mol, bevorzugt von 400 bis 6000 g/mol und besonders bevorzugt von 600 bis 3000 g/mol eingesetzt. Diese weisen bevorzugt eine OH-Funktionalität von 1,5 bis 6, besonders bevorzugt von 1,8 bis 3, ganz besonders bevorzugt von 1,9 bis 2,1 auf.

Geeignete polymere Polyole sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können einzeln oder in beliebigen Mischungen untereinander eingesetzt werden.

Geeignete Polyesterpolyole sind Polykondensate aus Di- sowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Butandiol(1,4), Neopentylglykol und Hydroxypivalinsäureneopentylglykolester bevorzugt sind. Daneben können auch Polyole wie Trimethylolpropan, Glycerin, Erythrit, Pentaerythrit, Trimetylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbemsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und Phthalsäure. Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Geeignete Polycarbonatpolyole sind Hydroxylgruppen-aufweisende Polycarbonate, bevorzugt Polycarbonatdiole, mit zahlenmittleren Molekulargewichten Mₙ von 400 bis 8000 g/mol, bevorzugt 600 bis 3000 g/mol. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und Lacton-modifizierte Diole der vorstehend genannten Art.

Bevorzugt enthält die Diolkomponente 40 bis 100 Gew.-% Hexandiol, bevorzugt sind 1,6-Hexandiol und/oder Hexandiolderivate. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole eingesetzt werden.

Hydroxylgruppen aufweisende Polycarbonate sind bevorzugt linear gebaut.

Geeignete Polyetherpolyole sind beispielsweise Polytetramethylenglykolpolyether, die durch Polymerisation von Tetrahydrofuran mittels kationischer Ringöffnung erhältlich sind.

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol.

Bevorzugte Polyole a2) sind Polytetramethylenglykolpolyether und Polycarbonat-Polyole bzw. deren Mischungen und besonders bevorzugt sind Polytetramethylenglykolpolyether.

Als hydroxyfunktionelle Verbindungen a3) können Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), Trimethylolpropan, Trimethylolethan, Glycerin, Pentaerythrit sowie deren beliebige Mischungen untereinander eingesetzt werden.

Geeignet sind auch Esterdiole des genannten Molekulargewichtsbereichs wie α-Hydroxybutylbutyl-ε-hydroxy-capronsäureester, ω-Hydroxyhexyl-y-hydroxybuttersäure-ester, Adipinsäure-(ß-hydroxyethyl)ester oder Terephthalsäurebis(ß-hydroxyethyl)-ester.

Ferner können auch monofunktionelle isocyanatreaktive Hydroxyl-gruppenhaltige Verbindungen eingesetzt werden. Beispiele solcher monofunktionellen Verbindungen sind Methanol, Ethanol, iso-Propanol, n-Propanol, n-Butanol, Ethylenglykolmonobutylether, Diethylenglykolmonomethylether, Diethylenglykolmonobutylether, Propylenglykolmonomethylether, Dipropylenglykol-monomethylether, Tripropylenglykolmonomethylether, Dipropylenglykolmono-propylether, Propylenglykolmonobutylether, Dipropylenglykolmonobutylether, Tripropylenglykolmonobutylether, 2-Ethylhexanol, 1-Octanol, 1-Dodecanol, 1-Hexadecanol. Falls derartige Alkohole mit dem Isocyanat-funktionellen Prepolymer regieren, werden die entsprechend abreagierten Anteile nicht mehr zu den Lösemitteln dazugezählt.

Als aminofunktionelle Verbindungen b1) können organische Di- oder Polyamine wie beispielsweise 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, 4,4-Diaminodicyclohexylmethan, und/oder Dimethylethylendiamin eingesetzt werden.

Darüber hinaus können auch aminofunktionelle Verbindungen b1) verwendet werden, die neben einer primären Aminogruppe auch sekundäre Aminogruppen oder neben einer Aminogruppe (primär oder sekundär) auch OH-Gruppen aufweisen. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1-methylaminobutan, Alkanolamine wie N-Aminoethylethanolamin, Ethanolamin, 3-Aminopropanol, Neopentanolamin.

Ferner können als aminofunktionelle Verbindungen b1) auch monofunktionelle Isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketim von diprimären Aminen, primär/tertiäre Amine, wie N,N-Dimethylaminopropylamin.

Bevorzugt werden 1,2-Ethylendiamin, Bis(4-aminocyclohexyl)methan, 1,4-Diaminobutan, Isophorondiamin, Ethanolamin, Diethanolamin und Diethylentriamin eingesetzt.

Bevorzugt werden die Komponenten a1), a2), a3) und b1) so gewählt, dass kein oder nur ein geringer Anteil an Verzweigungsstellen in dem Polyurethan gebildet wird, da ansonsten eine hohe Lösungsviskosität resultiert. Besonders bevorzugt werden ausschließlich Komponenten mit einer mittleren Funktionalität < 2,2 eingesetzt, ganz besonders bevorzugt mit einer mittleren Funktionalität < 2,05. In einer besonders bevorzugten Ausführungsform werden ausschließlich difunktionelle und monofunktionelle Bausteine eingesetzt und in einer ganz besonders bevorzugten Ausführungsform werden ausschließlich difunktionelle Bausteine eingesetzt.

In einer bevorzugten Ausführungsform werden die Komponenten a1) bis a3) und a1) in den folgenden Mengen zur Herstellung des Polyurethans eingesetzt, d.h. in das Polyurethan eingebaut, wobei sich die Einzelmengen stets zu 100 Gew.-% addieren:
5 bis 40 Gew.-% Komponente a1),
55 bis 90 Gew.-% Komponente a2),
0 bis 10 Gew.-% Komponente a3) und
1 bis 15 Gew.-% Komponente b1).

In einer besonders bevorzugten Ausführungsform werden die Komponenten a1) bis a3) und b1) in den folgenden Mengen zur Herstellung des Polyurethans eingesetzt, d.h. in das Polyurethan eingebaut, wobei sich die Einzelmengen stets zu 100 Gew.-% addieren:
5 bis 35 Gew.-% Komponente a1),
60 bis 85 Gew.-% Komponente a2),
0 bis 5 Gew.-% Komponente a3) und
3 bis 10 Gew.-% Komponente b1).

In einer ganz besonders bevorzugten Ausführungsform werden die Komponenten a1) bis a3) und b1) in den folgenden Mengen zur Herstellung des Polyurethans eingesetzt, d.h. in das Polyurethan eingebaut, wobei sich die Einzelmengen stets zu 100 Gew.-% addieren:
10 bis 30 Gew.-% Komponente a1),
65 bis 85 Gew.-% Komponente a2),
0 bis 3 Gew.-% Komponente a3) und
3 bis 8 Gew.-% Komponente b1).

Die vorangehend aufgeführten Mengen der einzelnen Komponenten a1), a2), a3) und b1) bezeichnen die zum Aufbau des Polyurethans eingesetzten Mengen und berücksichtigen nicht zusätzliche Mengen dieser Komponenten, die gegebenenfalls als Lösungsmittel vorhanden sind bzw. zugegeben werden.

Vor, während oder nach vollständig oder teilweise durchgeführter Polyaddition von a1), a2) und gegebenenfalls a3) kann ein Lösungsschritt erfolgen. Auch während oder nach Zugabe von b1) kann ein Lösungsschritt erfolgen.

Es sind Mischungen aus wenigstens zwei organischen Lösungsmitteln einsetzbar oder nur ein organisches Lösungsmittel. Bevorzugt sind Mischungen aus Lösungsmitteln.

Für die Herstellung von Polyurethan-Lösungen werden bevorzugt die Komponenten a1), a2) und gegebenenfalls a3) zur Herstellung eines isocyanatfunktionellen Polyurethan-Prepolymers ganz oder teilweise vorgelegt, gegebenenfalls mit einem gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden. Anschließend werden die gegebenenfalls zu Beginn der Reaktion noch nicht zugegebenen Bestandteile von a1), a2) und gegebenenfalls a3) zudosiert.

Bei der Herstellung des Polyurethan-Prepolymeren a) aus a1), a2) und gegebenenfalls a3) beträgt das Stoffmengenverhältnis von Isocyanat-Gruppen zu isocyanatreaktiven Gruppen im allgemeinen 1,05 bis 3,5, bevorzugt 1,1 bis 3,0, besonders bevorzugt 1,1 bis 2,5.

Unter isocyanatreaktiven Gruppen sind alle gegenüber Isocyanat-Gruppen reaktiven Gruppen zu verstehen, wie beispielsweise primäre und sekundäre Aminogruppen, Hydroxygruppen oder Thiolgruppen.

Die Umsetzung der Komponenten a1), a2) und gegebenenfalls a3) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Anschluss kann in einem weiteren Verfahrensschritt, falls noch nicht oder nur teilweise geschehen, das erhaltene Prepolymer mit Hilfe von einem oder mehreren organischen Lösemitteln gelöst werden.

Bei der Kettenverlängerung in Stufe b) werden NH₂- und/oder NH-funktionelle Komponenten mit den noch verbliebenen Isocyanatgruppen des Prepolymers umgesetzt.

Der Kettenverlängerungsgrad, also das Äquivalentverhältnis von NCO-reaktiven Gruppen der zur Kettenverlängerung und Kettenterminierung eingesetzten Verbindungen unter b) zu freien NCO-Gruppen des unter a) hergestellten Prepolymers, liegt im allgemeinen zwischen 50 und 150 %, bevorzugt zwischen 50 und 120 %, besonders bevorzugt zwischen 60 und 100 % und ganz besonders bevorzugt um 100%.

Die aminischen Komponenten b1) können gegebenenfalls in lösungsmittelverdünnter Form einzeln oder in Mischungen eingesetzt werden, wobei grundsätzlich jede Reihenfolge der Zugabe möglich ist. Auch alkoholische Lösemittel können zur Kettenverlängerung oder Kettenterminierung eingesetzt werden. Dabei wird in der Regel nur ein Teil der enthaltenen alkoholischen Lösemittel in die Polymerkette eingebaut.

Wenn organische Lösungsmittel als Verdünnungsmittel mit verwendet werden, so beträgt der Verdünnungsmittelgehalt in der in b) eingesetzten Komponente zur Kettenverlängerung bevorzugt 1 bis 95 Gew.-%, besonders bevorzugt 3 bis 50 Gew.%, bezogen auf das Gesamtgewicht der Komponente B1) einschließlich Verdünnungsmittel.

Typischerweise enthalten die verdünnten Polyurethan-Lösungen mindestens 5 Gew.-% Polyurethan, bezogen auf den Feststoffanteil aller in der Zusammensetzung enthaltenen Komponenten, d.h. bezogen auf den Gesamtfeststoffgehalt. Bevorzugt sind jedoch mindestens 30 Gew.-%, besonders bevorzugt mindestens 60 Gew.-%, und ganz besonders bevorzugt 70 bis 99 Gew.-% Polyurethan bezogen auf den Gesamtfeststoffgehalt enthalten.

Geeignete Lösungsmittel für die Polyurethan-Lösungen sind beispielsweise Ester, wie z.B. Ethylacetat oder Methyoxypropylacetat oder Butyrolacton, Alkohole, wie z.B. Ethanol, n-Propanol oder Isopropanol, Ketone, wie z.B. Aceton oder Methylethylketon, Ether, wie z.B. Tetrahydrofuran oder tert.-Butylmethylether. Bevorzugt werden Ester, Alkohole, Ketone und/oder Ether eingesetzt. Besonders bevorzugt ist zumindest ein Alkohol, bevorzugt zumindest ein aliphatischer Alkohol, besonders bevorzugt zumindest ein aliphatischer Alkohol mit 2 bis 6 Kohlenstoffatomen, wie beispielsweise Ethanol, n-Propanol und/oder Isopropanol enthalten und zumindest ein weiteres Lösemittel ausgewählt aus den Gruppen der Ester, Ketone oder Ether. Der besonders bevorzugte Gehalt alkoholischer Lösungsmittel beträgt 10 bis 80 Gew.%, ganz besonders bevorzugt 25 bis 65 Gew.%, bezogen auf das Gesamtgewicht aller Lösungsmittel. Alkohole werden im Rahmen der Erfindung als Lösungsmittel bezeichnet, solange sie nach Ausbildung des isocyanatfunktionellen Prepolymeren zugegeben werden. Der Anteil von Alkoholen, der als hydroxyfunktionelle Verbindung a3) bei der Herstellung des isocyanatfunktionellen Prepolymeren eingesetzt und in dieses kovalent eingebaut wird, zählt nicht zu den Lösungsmitteln.

Bevorzugt enthält die Polyurethan-Lösung weniger als 5 Gew.-%, bevorzugt weniger als 1 Gew.-%, besonders bevorzugt weniger als 0,3 Gew.-% Wasser bezogen auf das Gesamtgewicht der Lösung.

Zur Herstellung der Schutzschicht können auch Mischungen von verschiedenen Polymeren eingesetzt werden. Geeignet sind z.B. Mischungen aus Polymeren auf Basis von Polyurethanen, Polyestern, Poly(meth)acrylaten, Polyepoxiden, Polyvinylacetaten, Polyethylen, Polystyrol, Polybutadienen, Polyvinylchlorid und/oder entsprechenden Copolymerisaten.

Die zur Herstellung der Schutzschicht verwendbaren Polymere können zusätzlich auch Hilfs- und Zusatzstoffe enthalten. Beispiele für solche Hilfs- und Zusatzstoffe sind Vemetzter, Verdicker, Colösemittel, Thixotropiermittel, Stabilisatoren, Antioxidantien, Lichtschutzmittel, Weichmacher, Pigmente, Füllstoffe, Hydrophobierungsmittel und Verlaufshilfsmittel.

Die Polymere können zusätzlich Biozide, wundheilungs- fördernde oder andere Wirkstoffe, beispielsweise Analgetika oder Antiphlogistika enthalten.

Der Auftrag der Polymere, beispielsweise in From einer Lösung, kann nach allen an sich bekannten Applikationsformen erfolgen, genannt seien beispielsweise Rakeln, Streichen, Gießen oder Sprühen. Bevorzugt ist das Sprühen einer Lösung der Polymere.

Ein mehrschichtiger Auftrag mit gegebenenfalls zwischengelagerten Trocknungsschritten ist grundsätzlich auch möglich.

Die Schutzschicht aus den Polymeren kann nach dem Trocknen typischerweise eine Dicke von 1 bis 500 µm, bevorzugt 2 bis 300 µm, besonders bevorzugt 5 bis 200 µm, ganz besonders bevorzugt 5 bis 50 µm aufweisen.

Ein weiterer Gegenstand der Erfindung ist ein Verfahren zur Herstellung eines erfindungsgemäßen Klebstoffverbundsystems bei dem
I. auf ein Substrat eine Kleberschicht aus dem Gewebekleber und
II. auf die Klebeschicht flächig die wasserundurchlässige Schutzschicht aufgetragen wird.

Ebenfalls Gegenstand der Erfindung ist ein nach dem erfindungsgemäßen Verfahren erhältliches Klebstoffverbundsystem.

Gegenstand der Erfindung ist auch erfindungsgemäße Klebstoffverbundsystem zur Verwendung als Mittel zum Abdecken, Verschließen oder Verkleben von Zellgewebe

Auch die Verwendung eines erfindungsgemäßen Klebstoffverbundsystems zur Herstellung eines Mittels zum Abdecken, Verschließen oder Verkleben von Zellgewebe ist Gegenstand der Erfindung.

### Beispiele:

Die Erfindung wird im Folgenden detailliert anhand von Beispielen näher erläutert.

Sofern nicht abweichend gekennzeichnet, beziehen sich alle Prozentangaben auf das Gewicht.

Sofern nicht abweichend vermerkt, beziehen alle analytischen Messungen auf Temperaturen von 23°C.

Die Bestimmung der Festkörpergehalte erfolgte nach DIN-EN ISO 3251.

NCO-Gehalte wurden, wenn nicht ausdrücklich anders erwähnt, volumetrisch gemäß DIN-EN ISO 11909 bestimmt.

Die Kontrolle auf freie NCO-Gruppen wurde mittels IR-Spektroskopie (Bande bei 2260 cm ¹) durchgeführt.

Die angegebenen Viskositäten wurden mittels Rotationsviskosimetrie nach DIN 53019 bei 23°C mit einem Rotationsviskosimeter der Firma Anton Paar Germany GmbH, Ostfildern, DE bestimmt.

### Verwendete Substanzen und Abkürzungen:

| | |
|---|---|
| Desmophen^{®} C2200: | Polycarbonatpolyol, OH-Zahl 56 mg KOH/g, zahlenmittleres Molekulargewicht 2000 g/mol (Bayer MaterialScience AG, Leverkusen, DE) |
| Desmophen^{®} C1200: | Polycarbonatpolyol, OH-Zahl 112 mg KOH/g, zahlenmittleres Molekulargewicht 1000 g/mol (Bayer MaterialScience AG, Leverkusen, DE) |
| PoIyTHF^{®} 2000: | Polytetramethylenglykolpolyol, OH-Zahl 56 mg KOH/g, zahlenmittleres Molekulargewicht 2000 g/mol (BASF AG, Ludwigshafen, DE) |
| PoIyTHF^{®} 1000: | Polytetramethylenglykolpolyol, OH-Zahl 112 mg KOH/g, zahlenmittleres zahlenmittleres Molekulargewicht 1000 g/mol (BASF AG, Ludwigshafen, DE) |
| Desmophen NH1220 | Aminischer Härter, Equivalentgewicht 234 (Bayer Material Science AG) |

### Beispiel 1: Polymerschichten aus Polyurethan-Lösung (erfindungsgemäß)

200 g PoIyTHF^{®} 2000 und 50 g PoIyTHF^{®} 1000 wurden in einer Standard-Rührapparatur auf 80°C aufgeheizt. Anschließend wurde bei 80 °C innerhalb von 5 min ein Gemisch aus 66,72 g Isophorondiisocyanat und 520 g Methylethylketon zugegeben und am Rückfluss so lange gerührt (ca. 8 Stunden), bis der theoretische NCO-Wert erreicht war. Das fertige Prepolymer wurde auf 20°C abgekühlt und anschließend wurde eine Lösung aus 25,2 Methylenbis(4-aminocyclohexan) und 519,5 g Isopropanol innerhalb von 30 min zudosiert. Dann wurde weiter gerührt, bis IR-spektroskopisch keine freien Isocyanatgruppen mehr feststellbar waren.

Die erhaltene klare Lösung hatte nachfolgende Eigenschaften:

| | |
|---|---|
| Feststoffgehalt: | 25 % |
| Viskosität (Viskosimeter, 23°C): | 4600 mPas |

### Beispiel 2: Polymerschichten aus Polyurethan-Lösung (erfindungsgemäß)

200 g Desmophen C 2200 und 50 g Desmophen C 1200 wurden in einer Standard-Rührapparatur auf 80°C aufgeheizt. Anschließend wurde bei 80 °C innerhalb von 5 min ein Gemisch aus 66,72 g Isophorondiisocyanat und 520 g Methylethylketon zugegeben und am Rückfluss so lange gerührt (ca. 8 Stunden), bis der theoretische NCO-Wert erreicht war. Das fertige Prepolymer wurde auf 20°C abgekühlt und anschließend wurde eine Lösung aus 25,2 Methylenbis(4-aminocyclohexan) und 519,5 g Isopropanol innerhalb von 30 min zudosiert. Dann wurde weiter gerührt, bis IR-spektroskopisch keine freien Isocyanatgruppen mehr feststellbar waren.

Die erhaltene klare Lösung hatte nachfolgende Eigenschaften:

| | |
|---|---|
| Feststoffgehalt: | 26 % |
| Viskosität (Viskosimeter, 23°C): | 1800 mPas |

### Beispiel 3: Polymerschichten aus Polyurethan-Lösung (erfindungsgemäß)

225 g PolyTHF^{®} 2000 und 37,5 g PolyTFHF^{®} 1000 wurden in einer Standard-Rührapparatur auf 80°C aufgeheizt. Anschließend wurde bei 80 °C innerhalb von 5 min ein Gemisch aus 50,04 g Isophorondiisocyanat und 485 g Methylethylketon zugegeben und am Rückfluss so lange gerührt (ca. 16 Stunden, nach 8 Stunden Zusatz von 2 Tropfen DBTL), bis der theoretische NCO-Wert erreicht war. Das fertige Prepolymer wurde auf 20°C abgekühlt und anschließend wurde eine Lösung aus 13,70 g Methylenbis(4-aminocyclohexan) und 485 g Isopropanol innerhalb von 30 min zudosiert. Dann wurde weiter gerührt, bis IR-spektroskopisch keine freien Isocyanatgruppen mehr feststellbar waren und anschließend mit einer 1:1-Mischung aus Methylethylketon und Isopropanol ein Festgehalt von ca. 20 Gew.% eingestellt..

Die erhaltene klare Lösung hatte nachfolgende Eigenschaften:

| | |
|---|---|
| Feststoffgehalt: | 20,8 % |
| Viskosität (Viskosimeter, 23°C): | 11200 mPas |

### Beispiel 4: Polymerschichten aus Polyurethan-Lösung (erfindungsgemäß)

200 g PolyTHF^{®} 2000 und 50 g PolyTHF^{®} 1000 wurden in einer Standard-Rührapparatur auf 80°C aufgeheizt. Anschließend wurde bei 80C innerhalb von 5 min ein Gemisch aus 66,72 g Isophorondiisocyanat und 500 g Ethylacetat zugegeben und am Rückfluss so lange gerührt (ca. 8 Stunden), bis der theoretische NCO-Wert erreicht war. Das fertige Prepolymer wurde auf 20°C abgekühlt und anschließend wurde eine Lösung aus 31,3 g Methylenbis(4-aminocyclohexan) und 500g Isopropanol innerhalb von 30 min zudosiert. Dann wurde weiter gerührt, bis IR-spektroskopisch keine freien Isocyanatgruppen mehr feststellbar waren.

Die erhaltene klare Lösung hatte nachfolgende Eigenschaften:

| | |
|---|---|
| Feststoffgehalt: | 25% |
| Viskosität (Viskosimeter, 23°C): | 4600 mPas |

### Beispiel 5: Synthese eines stark quellenden Polyurethan-Wundklebers, der für die nachfolgenden Versuche eingesetzt wurde

92,6 g HDI und 0,25 g Dibutylphosphat wurden in einem 500 ml Vierhalskolben vorgelegt. Innerhalb von 2h wurden bei 80°C 157,1 g eines difunktionellen Polyethers mit einem Ethylenoxidgehalt von 71% und einem Propylenoxidgehalt von 29% (jeweils bezogen auf dem gesamten Alkylenoxidgehalt) hinzugefügt und 1h nachgerührt. Anschließend wurde durch Dünnschichtdestillation bei 130°C und 0,13 mbar das überschüssige HDI abdestilliert. Man erhielt das Prepolymers mit einem NCO-Gehalt von 2,42%. Der Restmonomerengehalt betrug < 0,03 % HDI. Viskosität: 2077 mPas.

### Beispiel 6: Beispiel eines ungeschützten Polyurethan-Wundklebers

4 g des Prepolymers aus Beispiel 5 wurden mit 0,53 g Desmophen NH1220 und 0,47 g PEG 200 mit Hilfe eines 2-Komponentenapplikators der Firma Medmix auf einer Glasplatte als 3 cm lange und 1cm breite Streifen aufgetragen. Die Platte wurde 20 min in 40°C warmes Wasser gestellt. Es hat eine vollständige Ablösung des Klebstoffes stattgefunden.

### Beispiel 7: Anwendungstechnische Beispiele für PUR-Lösungen

Auf einer Glasplatte wurden mit Hilfe eines Applikators 3 cm lange und 1 cm breite Streifen des Polyurethan-Wundklebers aus Beispiel 6 aufgetragen. Nach 30 min wurden die Polyurethan-Lösungen aus Beispiel 1-4 mit Hilfe eines Pinsels so aufgetragen, dass der Wundkleber sowie die umliegende Glasplatte vollständig bedeckt waren. Nach einer Trocknungszeit von 5 min. wurde die Platte 6 bis 40 min lang in bis zu 40°C warmes Wasser eingelegt und das Verhalten des Wundklebers hinsichtlich Quellung und/oder Ablösung von der Glasplatte untersucht. Unter den beschriebenen Polyurethan-Schutzfilmen blieb der Wundkleber bis zu 30 min bei 40°C optisch unverändert. Es hat keine Ablösung von der Glasplatte stattgefunden.

### Beispiel 8: Anwendungstechnisches Beispiel für ein Polyacrylat-System

Analog zu Beispiel 7 wurde der Wundkleber aus Beispiel 6 mit einem Acrylat-basierten Sprühpflaster, bestehend aus Polyisobuten, Isopropylhydrogenmaleat, Methylacrylat, Ethyl acetat und Pentan übersprüht. Der Schutzfilm hat bis zu 40 min bei 40°C den darunterliegenden Klebstoff vor Quellung geschützt.

## Patentansprüche

1. Klebstoffverbundsystem umfassend eine Kleberschicht aus einem Gewebekleber und eine flächig auf der Kleberschicht aufgebrachte Schutzschicht, wobei der Gewebekleber auf hydrophilen Polyurethanpolymeren basiert und die Schutzschicht wasserundurchlässig ist, **dadurch gekennzeichnet, dass** der Gewebekleber
A) isocyanatfunktionelle Prepolymere erhältlich aus
A1) aliphatischen Isocyanaten und
A2) Polyolen mit zahlenmittleren Molekulargewichten von ≥ 400 g/mol und mittleren OH-Funktionalitäten von 2 bis 6
B) aminofunktionelle Asparaginsäureester der allgemeinen Formel (I) 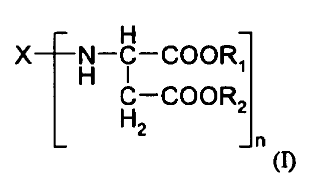 bei der
X ein n-wertiger organischer Rest ist, der durch Entfernung einer primären Aminogruppen eines n-wertigen Amins erhalten wird,
R₁, R₂ gleiche oder verschiedene organische Reste sind, die keinen Zerewitinoff- aktiven Wasserstoff aufweisen und
n eine ganze Zahl von mindestens 2 ist.
und / oder
C) Umsetzungsprodukte isocyanatfunktioneller Prepolymere A) mit Asparaginsäureestern B)
umfasst.

2. Klebstoffverbundsystem nach Anspruch 1, **dadurch gekennzeichnet, dass** die Isocyanate A1) ausschließlich aliphatisch oder cycloaliphatisch gebundene Isocyanatgruppe auf weisen.

3. Klebstoffverbundsystem nach einem der Ansprüche 1 oder 2, **dadurch gekennzeichnet, dass** die Polyole A2) Polyalkylenoxid-Polyether sind, die insbesondere einen Gehalt an Ethylenoxid-basierten Einheiten von 60 bis 90 Gew.-%, bezogen auf die insgesamt enthaltene Mengen an Alkylenoxideinheiten, aufweisen.

4. Klebstoffverbundsystem nach einem der Ansprüche 1 bis 3, **dadurch gekennzeichnet, dass** die Asparaginsäureester B) Verbindungen gemäß der Formel (I) sind, bei denen
X von 4-Diaminobutan, 1,5-Diaminopentan, 2-Methyl-1,5-diaminopentan, 1,6-Diaminohexan, 2,2,4- oder 2,4,4-Trimethyl-1,6-diaminohexan als n-wertigen Aminen abgeleitet ist,
R₁, R₂ jeweils unabhängig voneinander ein C₁ bis C₁₀-Alkylrest sind und
n = 2 ist.

5. Klebstoffverbundsystem nach einem der Ansprüche 1 bis 4, **dadurch gekennzeichnet, dass** der Gewebekleber keinen Asparaginsäureester B) sondern ausschließlich Umsetzungsprodukte C) umfasst.

6. Klebstoffverbundsystem nach einem der Ansprüche 1 bis 5, **dadurch gekennzeichnet, dass** die Schutzschicht eine Bruchdehnung von ≥ 100%, bevorzugt von ≥ 200% auf weist.

7. Klebstoffverbundsystem nach einem der Ansprüche 1 bis 6, **dadurch gekennzeichnet, dass** die Schutzschicht einen 100%-Modul von 0,5 bis 20 MPa, bevorzugt von 1 bis 15 MPa, besonders bevorzugt von 2 bis 10 MPa aufweist.

8. Klebstoffverbundsystem nach einem der Ansprüche 1 bis 7, **dadurch gekennzeichnet, dass** die Schutzschicht auf Polymeren basiert.

9. Klebstoffverbundsystem nach Anspruch 8, **dadurch gekennzeichnet, dass** die Polymere Polyurethane, Polyester, Poly(meth)acrylate, Polyepoxide, Polyvinylacetate, Polyethylene, Polystyrole, Polybutadiene, Polyvinylchloride und / oder entsprechenden Copolymerisaten, bevorzugt Polyacrylate und / oder Polyurethane sind.

10. Klebstoffverbundsystem nach Anspruch 9, **dadurch gekennzeichnet, dass** die Polymere Polyurethane sind, die nach einem Prepolymerisationverfahren erhältlich sind, bei dem
a) isocyanatfunktionelle Prepolymere aus
a1) organischen Polyisocyanaten
a2) polymeren Polyolen mit zahlenmittleren Molekulargewichten von 400 bis 8000 g/mol, vorzugsweise 400 bis 6000 g/mol und besonders bevorzugt von 600 bis 3000 g/mol, und OH-Funktionalitäten von 1,5 bis 6, bevorzugt 1,8 bis 3, besonders bevorzugt von 1,9 bis 2,1, und
a3) gegebenenfalls hydroxyfunktionellen Verbindungen mit Molekulargewichten von 62 bis 399 g/mol
hergestellt werden, und
b) die freien NCO-Gruppen der Prepolymeren aus a) dann ganz oder teilweise mit
b1) aminofunktionellen und/oder hydroxyfunktionellen Verbindungen mit Molekulargewichten von 32 bis 1000 g/mol, bevorzugt von 32 bis 400 g/mol
unter Kettenverlängerung umgesetzt werden.

11. Verfahren zur Herstellung eines Klebstoffverbundsystems nach einem der Ansprüche 1 bis 10, bei dem
I. auf ein Substrat eine Kleberschicht aus dem Gewebekleber und
II. auf die Klebeschicht flächig die wasserundurchlässige Schutzschicht aufgetragen wird.

12. Klebstoffverbundsystem erhältlich nach dem Verfahren des Anspruchs 11.

13. Klebstoffverbundsystem nach einem der Ansprüche 1 bis 10 oder 12 zur Verwendung als Mittel zum Abdecken, Verschließen oder Verkleben von Zellgewebe.

14. Verwendung eines Klebstoffverbundsystems nach einem der Ansprüche 1 bis 10 oder 12 zur Herstellung eines Mittels zum Abdecken, Verschließen oder Verkleben von Zellgewebe.

## Claims

1. Composite adhesive system comprising an adhesive layer composed of a tissue adhesive, and a protective layer applied extensively over the adhesive layer, the tissue adhesive being based on hydrophilic polyurethane polymers, and the protective layer being water-impermeable, **characterized in that** the tissue adhesive comprises
A) isocyanate-functional prepolymers obtainable from
A1) aliphatic isocyanates and
A2) polyols having number-average molecular weights of ≥ 400 g/mol and average OH functionalities of 2 to 6,
B) amino-functional aspartic esters of the general formula (I) 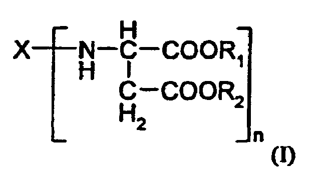 in which
X is an n-valent organic radical obtained by removing a primary amino group of an n-valent amine,
R₁ and R₂ are identical or different organic radicals which contain no Zerewitinoff- active hydrogen, and
n is an integer of at least 2,
and/or
C) reaction products of isocyanate-functional prepolymers A) with aspartic esters B).

2. Composite adhesive system according to Claim 1, **characterized in that** the isocyanates A1) contain exclusively aliphatically or cycloaliphatically bonded isocyanate group.

3. Composite adhesive system according to either of Claims 1 and 2, **characterized in that** the polyols A2) are polyalkylene oxide polyethers having more particularly an ethylene oxide-based units content of 60% to 90% by weight, based on the amounts of alkylene oxide units present overall.

4. Composite adhesive system according to any of Claims 1 to 3, **characterized in that** the aspartic esters B) are compounds of the formula (I) in which
X derives from 4-diaminobutane, 1,5-diaminopentane, 2-methyl-1,5-diaminopentane, 1,6-diaminohexane, 2,2,4- or 2,4,4-trimethyl-1,6-diaminohexane as n-valent amines,
R₁ and R₂ each independently of one another are a C₁ to C₁₀-alkyl radical, and
n is 2.

5. Composite adhesive system according to any of Claims 1 to 4, **characterized in that** the tissue adhesive comprises no aspartic ester B) but instead exclusively reaction products C).

6. Composite adhesive system according to any of Claims 1 to 5, **characterized in that** the protective layer has an elongation at break of ≥ 100%, preferably of ≥ 200%.

7. Composite adhesive system according to any of Claims 1 to 6, **characterized in that** the protective layer has a 100% modulus of 0.5 to 20 MPa, preferably of 1 to 15 MPa, more preferably of 2 to 10 MPa.

8. Composite adhesive system according to any of Claims 1 to 7, **characterized in that** the protective layer is based on polymers.

9. Composite adhesive system according to Claim 8, **characterized in that** the polymers are polyurethanes, polyesters, poly(meth)acrylates, polyepoxides, polyvinyl acetates, polyethylenes, polystyrenes, polybutadienes, polyvinyl chlorides and/or corresponding copolymers, preferably polyacrylates and/or polyurethanes.

10. Composite adhesive system according to Claim 9, **characterized in that** the polymers are polyurethanes which are obtainable by a prepolymerization process in which
a) isocyanate-functional prepolymers are prepared from
a1) organic polyisocyanates,
a2) polymeric polyols having number-average molecular weights of 400 to 8000 g/mol, preferably 400 to 6000 g/mol and more preferably of 600 to 3000 g/mol, and OH functionalities of 1.5 to 6, preferably 1.8 to 3, more preferably of 1.9 to 2.1, and
a3) optionally hydroxyl-functional compounds having molecular weights of 62 to 399 g/mol,
and
b) the free NCO groups of the prepolymers from a) are then reacted wholly or partly, with chain extension, with
b1) amino-functional and/or hydroxyl-functional compounds having molecular weights of 32 to 1000 g/mol, preferably of 32 to 400 g/mol.

11. Method for producing a composite adhesive system according to any of Claims 1 to 10, wherein
I. an adhesive layer composed of the tissue adhesive is applied to a substrate and
II. the water-impermeable protective layer is applied extensively to the adhesive layer.

12. Composite adhesive system obtainable by the method of Claim 11.

13. Composite adhesive system according to any of Claims 1 to 10 and 12 for use as a means of covering, sealing or bonding cell tissue.

14. Use of a composite adhesive system according to any of Claims 1 to 10 and 12 for producing a means for covering, sealing or bonding cell tissue.

## Revendications

1. Système composite d'adhésif comprenant une couche d'adhésif à base d'une colle pour tissu et une couche protectrice appliquée sur toute la surface de la couche d'adhésif, la colle pour tissu étant à base de polymères polyuréthane hydrophiles et la couche protectrice étant imperméable à l'eau, **caractérisé en ce que** la colle pour tissu comprend
A) des prépolymères à fonction isocyanate pouvant être obtenus à partir
A1) d'isocyanates aliphatiques et
A2) de polyols ayant des masses moléculaires moyennes en nombre de ≥ 400 g/mole et des fonctionnalités OH moyennes de 2 à 6
B) des esters d'acide aspartique à fonction amino, de formule générale (I) 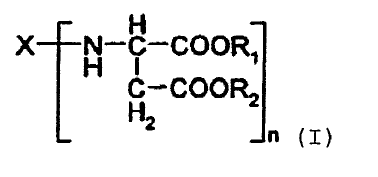 dans laquelle
X est un radical organique n-valent, qui est obtenu par enlèvement d'un groupe amino primaire d'une amine n-valente,
R₁, R₂ représentent des radicaux organiques identiques ou différents, qui ne comportent pas d'atomes d'hydrogène actifs selon la méthode de Zerewitinoff et
n est un nombre entier valant au moins 2,
et/ou
C) des produits de réaction de prépolymères à fonction isocyanate A) avec des esters d'acide aspartique B).

2. Système composite d'adhésif selon la revendication 1, **caractérisé en ce que** les isocyanates A1) comportent exclusivement un groupe isocyanate en liaison aliphatique ou cycloaliphatique.

3. Système composite d'adhésif selon la revendication 1 ou 2, **caractérisé en ce que** les polyols A2) sont des polyoxyalkylène-polyéthers qui ont en particulier une teneur en motifs à base d'oxyde d'éthylène de 60 à 90 % en poids, par rapport aux quantités de motifs oxyde d'alkylène contenues au total.

4. Système composite d'adhésif selon l'une quelconque des revendications 1 à 3, **caractérisé en ce que** les esters d'acide aspartique B) sont des composés de formule (I), dans lesquels
X est dérivé du 4-aminobutane, 1,5-diaminopentane, 2-méthyl-1,5-diaminopentane, 1,6-diaminohexane, 2,2,4- ou 2,4,4-triméthyl-1,6-diaminohexane en tant qu'amines n-valentes,
R₁, R₂ représentent chacun, indépendamment l'un de l'autre, un radical alkyle en C₁-C₁₀ et
n = 2.

5. Système composite d'adhésif selon l'une quelconque des revendications 1 à 4, **caractérisé en ce que** la colle pour tissu ne comprend pas d'esters d'acide aspartique B) mais exclusivement des produits de réaction C).

6. Système composite d'adhésif selon l'une quelconque des revendications 1 à 5, **caractérisé en ce que** la couche protectrice présente un allongement à la rupture de ≥ 100 %, de préférence de ≥ 200 %.

7. Système composite d'adhésif selon l'une quelconque des revendications 1 à 6, **caractérisé en ce que** la couche protectrice présente un module d'élasticité à 100 % de 0,5 à 20 MPa, de préférence de 1 à 15 MPa, de façon particulièrement préférée de 2 à 10 MPa.

8. Système composite d'adhésif selon l'une quelconque des revendications 1 à 7, **caractérisé en ce que** la couche protectrice est à base de polymères.

9. Système composite d'adhésif selon la revendication 8, **caractérisé en ce que** les polymères sont des polyuréthanes, polyesters, poly(méth)-acrylates, polyépoxydes, poly(acétate de vinyle)s, polyéthylènes, polystyrènes, polybutadiènes, poly(chlorure de vinyle)s et/ou des copolymères correspondants, de préférence des polyacrylates et/ou des polyuréthanes.

10. Système composite d'adhésif selon la revendication 9, **caractérisé en ce que** les polymères sont des polyuréthanes qui peuvent être obtenus selon un procédé de prépolymérisation, dans lequel on prépare
a) des prépolymères à fonction isocyanate à partir
a1) de polyisocyanates organiques
a2) de polyols polymères ayant des masses moléculaires moyennes en nombre de 400 à 8 000 g/mole, de préférence de 400 à 6 000 g/mole et de façon particulièrement préférée de 600 à 3 000 g/mole, et des fonctionnalités OH de 1,5 à 6, de préférence de 1,8 à 3, de façon particulièrement préférée de 1,9 à 2,1, et
a3) éventuellement de composés à fonction hydroxy ayant des masses moléculaires de 62 à 399 g/mole,
et
b) on fait ensuite réagir les groupes NCO libres des prépolymères de a) en partie ou en totalité avec
b1) des composés à fonction amino et/ou à fonction hydroxy ayant des masses moléculaires de 32 à 1 000 g/mole, de préférence de 32 à 400 g/mole
avec extension de chaîne.

11. Procédé pour la préparation d'un système composite d'adhésif selon l'une quelconque des revendications 1 à 10, dans lequel
I. on applique sur un support une couche d'adhésif à base de la colle pour tissu et
II. on applique sur toute la surface de la couche d'adhésif la couche protectrice imperméable à l'eau.

12. Système composite d'adhésif pouvant être obtenu selon le procédé de la revendication 11.

13. Système composite d'adhésif selon l'une quelconque des revendications 1 à 10 ou 12, pour utilisation comme produit pour le recouvrement, la fermeture ou le collage de tissu cellulaire.

14. Utilisation d'un système composite d'adhésif selon l'une quelconque des revendications 1 à 10 ou 12 pour la fabrication d'un produit destiné au recouvrement, à la fermeture ou au collage de tissu cellulaire.
